# EUROPEAN PATENT APPLICATION

(11) **EP 4 844 376 A1**
(43) Date of publication of application: **25.11.2026**
(21) Application number: 25177437.8
(22) Date of filing: 19.05.2025
(51) Int. Cl.: G01N 21/3563, G01N 21/359, G01N 21/47, G01N 21/49, G01N 33/02, G01J 3/02, G01J 3/10

(54) **A DEVICE FOR AND METHOD OF DETERMINING THE COMPOSITION OF A SAMPLE**

(71) Applicant: Nofima AS, 9291 Tromsø (NO); Sintef TTO AS, 7465 Trondheim (NO)
(72) Inventor: Tschudi, Jon, 0614 Oslo (NO); O'Farrell, Marion, 0614 Oslo (NO); Wold, Jens Petter, 9291 Tromso (NO)
(74) Representative: Dehns

(57) **Abstract**

A device (100) for determining the composition of a sample (110), including an illumination module (102), a detector (104), a guiding module (106) and a processor (126). The illumination module includes a first light source (101) that projects an illumination pattern (108) on the sample in a first direction. The detector measures light from the first light source scattered inside the sample into a field-of-view (111) of the detector. The guiding module includes a second light source (103) to project a guiding pattern (109) on the sample in a second direction. The position of the guiding pattern projected onto the sample selectively indicates when the device is positioned relative to the sample such that the field-of-view of the detector and the illumination pattern are within a first predetermined range.

## Description

This invention relates to a device for and a method of determining the composition of a sample, in particular using non-contact spectroscopy with a portable device.

Non-contact analysis of foodstuffs using non-destructive near-infrared interactance spectroscopy can be used to determine sub-surface composition, which can be useful for determining the contents and quality of produce such as fruit and meat. This can prevent produce contamination and the quality of the produce being deteriorated by destructive or damaging measurements, such as squeezing the legs of a crab to determine meat content, which can damage the legs and cause meat discolouration, or bruising of fruit when it is pressed, for example to determine water content or ripeness.

It is useful to use a portable device to take such measurements. However, it can be difficult to accurately position such a device (e.g. at a desired distance to a product for an accurate measurement to be taken), leading to incorrect determinations of the composition of produce, which could lead to the produce being incorrectly valued or thrown away.

The present invention aims to address this problem.

From a first aspect, the invention provides a device for determining the composition of a sample, the device comprising:
an illumination module comprising a first light source arranged to project an illumination pattern on the sample in a first direction;
a detector arranged to measure light from the first light source scattered inside the sample into a field-of-view of the detector;
a guiding module comprising a second light source arranged to project a guiding pattern on the sample in a second direction;
   wherein the guiding module is arranged to project the guiding pattern in the second direction at an angle relative to the first direction in which the illumination module projects the illumination pattern, such that the relative positions of the guiding pattern and the illumination pattern on the sample vary depending on the position of the device relative to the sample;
   wherein the detector is positioned at an angle relative to the illumination module, such that the relative positions of the field-of-view of the detector and the illumination pattern on the sample vary depending on the position of the device relative to the sample;
   wherein the guiding module, the illumination module and the detector are arranged such that the position of the guiding pattern projected onto the sample selectively indicates when the device is positioned relative to the sample such that the field-of-view of the detector and the illumination pattern are within a first predetermined range;
the device further comprising:
   a processor programmed to:
   operate the illumination module to project the illumination pattern;
   operate the guiding module to project the guiding pattern;
   operate the detector to take measurements;
   receive measurements from the detector; and
   output or store data representative of the measurements from the detector.

From a second aspect, the invention provides a computer-implemented method for determining the composition of a sample using a device comprising a processor, an illumination module, a guiding module, a detector and a camera, wherein the processor is programmed to control the illumination module, the guiding module, the detector and the camera;
wherein the processor is programmed to, when operating in a guiding mode:
   operate the illumination module to project an illumination pattern on the sample in a first direction;
   operate the guiding module to project a guiding pattern on the sample in a second direction at an angle relative to the first direction in which the illumination module projects the illumination pattern, such that the relative positions of the guiding pattern and the illumination pattern vary depending on the position of the device relative to the sample;
   receive an image from the camera of the illumination pattern and the guiding pattern; and
   determine the position of the guiding pattern on the sample;
wherein the processor is programmed to, when the processor is operating in an illumination mode:
   operate the detector to measure light scattered inside the sample into a field-of-view of the detector;
   receive measurements from the detector; and
   output or store data representative of the measurements from the detector;
   wherein the processor is programmed to switch from the guiding mode to the illumination mode when the processor determines that the guiding pattern is positioned relative to the sample such that the field-of-view of the detector and the illumination pattern are within a first predetermined range.

Thus it will be seen that, in accordance with embodiments of the invention, the device is arranged to be moved relative to the sample until the field-of-view of the detector and the illumination pattern are aligned on the surface of the sample (i.e. the detector field-of-view and the illumination pattern are within a first predetermined range). This can also assist the user in taking the measurement at the desired position on the sample. The light from the illumination pattern enters the sample, where it is scattered inside the sample, and then exits the sample towards the detector.

The field-of-view of the detector on the sample is the solid angle subtended from the detector projected onto a plane, e.g. projected onto the sample. This is the region on the sample from which the detector can detect light (i.e. a detection region). For example, the detector may detect light that emerges from the sample within the detection region, and may not detect light outside of this region. Thus, in use, the field-of-view of the detector covers a particular portion of the sample.

The illumination module is arranged at a non-parallel angle relative to the detector, and is arranged to project the illumination pattern in a first direction onto the sample, e.g. where the first direction is at an angle with respect to the (e.g. centre of the) solid angle subtended from the detector. The illumination module and the detector are arranged such that when the sample is placed at a given position (e.g. in a given plane) spaced from the device (i.e. where the given position is where it is desired to place the sample), the field-of-view of the detector and the illumination pattern are arranged on the sample within the first predetermined range.

Owing to the relative angles of the illumination module and the detector (e.g. the relative angles of the first direction and the (e.g. centre of the) solid angle subtended from the detector), when the sample is placed in a position too close or too far from the detector, the illumination pattern may not be within the first predetermined range of the field-of-view of the detector . For example, the illumination module may project light at an angle between 0 and 90 degrees relative to the (e.g. centre of the) solid angle subtended from the detector.

The guiding module is arranged to project a guiding pattern onto the sample in a second direction, where the second direction is also at a non-parallel angle relative to the first direction of projection of the illumination module (i.e. the guiding module and the illumination module are mounted in the device at angles with respect to one another). Thus, the position of the guiding pattern when projected on the sample indicates the distance of the device from the sample, and therefore whether the field-of-view of the detector and the illumination pattern are within the first predetermined range.

In some embodiments, the relative positions of the guiding pattern and the illumination pattern indicate that the device is positioned relative to the sample such that the field-of-view of the detector and the illumination pattern are within the first predetermined range on the sample (e.g. the sample is in the desired position relative to the device), when the relative positions of the guiding pattern and the illumination pattern are within a second predetermined range on the sample. For example, the device may be arranged to be moved relative to the sample until the guiding patterns and the illumination patterns are aligned on the surface of the sample.

When the guiding patterns and illumination patterns are aligned on the surface of the sample, the device may be positioned relative to the sample such that the field-of-view of the detector and the illumination pattern are within the first predetermined range on the sample. The illumination module and the guiding module are arranged to project the illumination pattern at an angle with respect to one another. Thus, the illumination pattern and the guiding pattern when projected on the sample may only be within the second predetermined range when the device is held at a particular distance from the sample. For example, the illumination module may project light an angle between 0 and 90 degrees relative to the direction in which the guiding module projects light.

In some embodiments, the solid angle subtended from the detector overlaps with the second direction, such that the position of the guiding pattern on the sample may indicate the field-of-view of the detector. For example, the first predetermined range may be substantially the same as the second predetermined range.

This is novel and inventive in its own right, and thus when viewed from a third aspect, the invention provides a positioning module for a device for determining the composition of a sample, comprising:
an illumination module comprising a first light source arranged to project an illumination pattern on the sample in a first direction;
a guiding module comprising a second light source arranged to project a guiding pattern on the sample in a second direction;
   wherein the guiding module is arranged to project the guiding pattern in the second direction at an angle relative to the first direction in which the illumination module projects the illumination pattern, such that the relative positions of the guiding pattern and the illumination pattern on the sample vary depending on the position of the device relative to the sample;
   wherein the illumination module and the guiding module are arranged such that the relative positions of the guiding pattern and the illumination pattern projected onto the sample selectively indicates when the device is positioned relative to the sample such that the relative positions of the guiding pattern and the illumination pattern are within a predetermined range on the sample.

It will be appreciated that the third aspect of the invention may be combined with the first and/or second aspects of the invention described above, e.g. the predetermined range of the third aspect may be a second predetermined range.

The device may be held by a user, mounted to a robot, and/or mounted onto a stand (e.g. above a conveyor belt). As the device is not in contact with the sample, the guiding pattern allows for the device to be positioned at the (e.g. desired) distance from the sample such that measurements can be taken by the detector when the device is at the desired distance from the sample.

For example, where the relative positions of the guiding pattern and the illumination pattern indicate that the device is in a desired position relative to the sample (e.g. the guiding pattern and the illumination pattern are within the second predetermined range), the illumination pattern may comprise a ring and the guiding pattern may comprise a circle smaller than the ring, and the desired position (e.g. the desired distance between the device and the sample) may be where the guiding pattern is in the centre of the illumination pattern on the surface of the sample. As the illumination pattern and guiding pattern are projected onto the surface of the sample, at least the guiding pattern may be seen by the user, e.g. to assist the user with positioning the device in the desired position relative to the sample.

In some embodiments, the device is arranged to receive an input from a user to take measurements, when the user determines that the relative positions of the guiding pattern and the illumination pattern are within the second predetermined range. Preferably, both the illumination pattern and the guiding pattern are visible to the user, e.g. the illumination module and/or the guiding module are arranged to emit light having at least a portion in the visible range.

The user may move the sample or the device, until they determine that the guiding pattern and the illumination distance are within the second predetermined range. For example, the user may determine that the guiding pattern is approximately in the centre of the illumination pattern and is thus in the second predetermined range. The user may then input to the device to start taking measurements, e.g. by pressing a button which indicates to the processor to acquire data from the detector.

In some embodiments, the processor is programmed to determine the stability of the measurements by comparing the light intensity of the measurements (e.g. over time). The processor may be programmed to output or store data representative of the measurements of stability. For example, when the measurements taken have a substantially constant overall intensity, the device may be stable, and the background light may be substantially constant.

However, when the intensity fluctuates significantly whilst measurements are being taken, the device may have moved during the measurement or the background light may have fluctuated, meaning that the measurements are unstable. When the processor determines that the measurements are unstable (e.g. the variation in the light intensity is above a threshold variation), the processor may discard the unstable measurements during processing of the measurements. For example, the processor may be programmed to determine the stability of the measurements during operation of the detector, e.g. to discard any unstable measurements during the measurement period that are below a threshold stability, e.g. above a threshold variation in light intensity.

In some embodiments the device comprises a camera arranged to image the guiding pattern;
wherein the processor is further arranged to:
receive an image from the camera; and
determine the position of the guiding pattern on the sample using the image from the camera.

The processor may be programmed to use the position of the guiding pattern in the image to determine whether the guiding pattern is in the desired position, and therefore whether the field-of-view of the detector and the illumination pattern are within the first predetermined range.

In some embodiments, the processor is programmed to determine the position of the guiding pattern on the sample using the image from the camera, wherein the position of the guiding pattern on the sample indicates when the device is positioned relative to the sample such that the position of the guiding pattern is within a predetermined position in the image captured by the camera. For example, the camera may be positioned at an angle relative to the guiding module, such that the relative position of the guiding pattern in the image captured by the camera may depend on the position of the device relative to the sample. This may result in the position and/or size of the guiding pattern in the image varying depending on the relative position of the device and the sample.

The processor may be programmed to determine whether the guiding pattern is within a predetermined position or range of positions in the image that indicates that the device is positioned relative to the sample such that the field-of-view of the detector and the illumination pattern are within a first predetermined range. For example, when the device and the sample are too close to one another, owing to the relative angle between the guiding module and the camera, the guiding pattern may appear to be higher than the predetermined position in the image, and vice versa.

In some embodiments, the camera is arranged to image the illumination pattern and the guiding pattern, and the processor is programmed to determine the relative positions of the illumination pattern and the guiding pattern on the sample using the image from the camera, wherein the relative positions of the guiding pattern and the illumination pattern indicate when the device is positioned relative to the sample such that the relative positions of the guiding pattern and the illumination pattern are within a predetermined range on the sample. For example, the processor may be programmed to determine the number of pixels between the guiding pattern and the illumination pattern in the image captured by the camera.

For example, the device may be arranged to indicate to the user, e.g. via a light or user interface, when the device is at the desired position (e.g. desired distance) relative to the sample, e.g. based on the position of the guiding pattern in the image, or the relative positions of the illumination pattern and the guiding pattern imaged by the camera. The user may then input to the device to start taking measurements. Furthermore, the camera may be used to identify and/or document the sample being analysed, e.g. such that the measurements taken by the detector may be easily associated with a particular sample.

In some embodiments, the processor is programmed to determine the stability of the measurements taken by comparing the positions of the guiding pattern in the image, and/or the relative distances of the guiding pattern and the illumination pattern on the sample (e.g. over time). The processor may be programmed to output or store data representative of the measurements of stability. The processor may discard measurements taken when the device was moving too much, as the detector may not be able to accurately measure the light scattered inside the sample, e.g. during and/or after the measurement period. For example, the processor may be programmed to output to the user (e.g. via a user interface) when the device is moving too much relative to the sample, to indicate to the user that they should hold the sample or device still.

In some embodiments, in a guiding mode the processor is programmed to:
operate the illumination module to project the illumination pattern and operate the guiding module to project the guiding pattern;
wherein the processor is programmed to, when the processor is operating in an illumination mode:
   operate the detector to take measurements;
   receive measurements from the detector; and
   output or store data representative of the measurements from the detector; and
wherein the processor is programmed to switch from the guiding mode to the illumination mode when the processor determines that the position of the guiding pattern indicates that the device is positioned relative to the sample such that the field-of-view of the detector and the illumination pattern are within the first predetermined range. This may allow for an accurate measurement to be quickly taken as soon as the device is detected to be in the desired position.

In a preferred embodiment, the processor is programmed to determine when the guiding pattern is in the desired position, such that the device is positioned relative to the sample such that the field-of-view of the detector and the illumination pattern are within the first predetermined range, e.g. when the guiding pattern is in the predetermined position in the image and/or the guiding pattern and the illumination pattern are within the second predetermined range on the sample.

The processor may be programmed to operate the detector to measure light when the it has determined that the guiding pattern is in the desired position. For example, when the patterns are not aligned or the guiding pattern is not in the desired position in the image, the device may be too close or too far from the sample to be able to perform a desired measurement of the scattered light, e.g. the light detected by the detector may not have scattered at the desired depth in the sample and/or the signal may be dominated by light reflected at the surface of the sample. For example, the detector may be arranged to have a field-of-view that can improve the signal from (e.g. maximises) the desired depth of the sample when the guiding pattern and the illumination pattern are aligned, e.g. the field-of-view of the detector on the sample may be arranged to be substantially the same as the as the area of the guiding pattern projected onto the sample. For example, the camera field-of-view may comprise the detector field-of-view.

In some embodiments, the processor is programmed to store measurements from the detector when the processor has determined that the guiding pattern is in the desired position, e.g. when the guiding pattern is in the predetermined position in the image and/or when the guiding pattern and the illumination pattern are within the second predetermined range. For example, the processor may be programmed to continuously operate the detector, and the measurements taken may be used in a preview mode, in order for the processor to verify whether the device is in the desired position relative to the device, e.g. in addition to the relative positions of the guiding pattern and the illumination pattern. For example, the measurements taken by the detector in the preview mode may be displayed on a user interface, where a user may determine whether the measurements are as expected, before starting to take formal measurements that are stored.

The processor may be programmed to be in the guiding mode when the device is turned on, in order to help ensure that the device is in the desired position before any measurements are taken. In some embodiments, the processor is programmed to operate the detector to stop taking measurements when the processor determines that the guiding pattern is in the predetermined position in the image and/or the relative positions of the guiding pattern and the illumination pattern are outside of the second predetermined range in the illumination mode. For example, the processor may be programmed to flag the data representative of measurements from the detector to indicate that the device was moved, and/or the processor may turn off the detector.

In some embodiments, the device is handheld. For example, a user may wish to analyse the composition of a strawberry, e.g. holding the device in one hand, and the strawberry in the other hand. The device and strawberry may be moved relative to one another, until the device is at the desired position relative to the strawberry in order for the detector to take a measurement of the light scattered inside the sample.

The handheld device may be used to measure the properties of a sample, using the light scattered inside the sample. This may enable samples, e.g. foodstuffs, to be analysed in-situ, without the need for large, fixed instrumentation (e.g. in a laboratory or factory setting). A non-contact device may prevent the sample being damaged during the measurement process, e.g. the bruising of fruit. This may also allow the device to be used with live produce, e.g. crabs, that may be moving.

The light from the illumination pattern is projected (shone) onto the sample, where it is absorbed, transmitted, scattered and/or reflected by the sample. The detector is arranged to measure the light from the first light source that is scattered inside the sample, e.g. the light that has interacted below the surface of the sample.

In some embodiments, the field-of-view of the detector (i.e. a detection region) is adjacent to and spaced from the illumination pattern on the sample, e.g. spaced (laterally) from the illumination pattern, e.g. when the guiding pattern is in the predetermined position in the image and/or when the relative positions of the guiding pattern and the illumination pattern are within the second predetermined range. Thus, the detector may be arranged to measure the light from the first light source that is internally scattered (e.g. reflected laterally) by the sample and has re-emerged in the detection region.

This may enable the detector to detect light from the first light source that has penetrated into the sample (e.g. 10 mm into the sample), e.g. the light that has interacted with matter below the surface of the sample by being scattered multiple times. By measuring the light that has interacted below the surface of the sample, the measurements taken by the detector can be used to determine the composition of the sample below the surface.

Preferably, the field-of-view of the detector (i.e. the detection region) does not overlap with the illumination pattern when the guiding pattern and illumination patterns are within the second predetermined range and/or when the position of the guiding pattern is in the predetermined position. This may prevent the detector from taking measurements of light that is directly reflected from the surface of the sample. For example, when the detection region is too close to the illumination pattern, the light detected by the detector may be dominated by light that is directly reflected from the surface of the sample, or light that has only partially penetrated the sample, e.g. very close to the surface.

In contrast, when the detection region is spaced from the illumination pattern, the detected light may be dominated by light that has interacted below the surface of the sample, as the light has generally scattered deeper into the sample before re-emerging at the detection region. The light that is reflected directly from the surface of the sample may be dominated by information regarding the surface composition of the sample, rather than the composition of the main body of the sample below the surface.

For example, if the device is not in the desired position relative to the sample (e.g. the guiding pattern and the illumination patterns are not in the second predetermined range), the detector may receive light from the first light source that has been reflected directly from the sample, and not scattered light, i.e. light that has interacted inside the sample before being detected. Thus, the relative positions of the guiding pattern and the illumination pattern may allow for the device to be positioned such that the detector is arranged take interactance measurements of the sample (when the guiding pattern and the illumination pattern are within the second predetermined range).

As the illumination pattern may be spaced from the detection region, the signal from photons that can reach the detection region may be relatively weak, e.g. when compared to the reflection of light from the surface of the sample. In order for light to be internally scattered from the illumination pattern to the detection region to determine the composition of the sample, the illumination pattern may comprise a large enough area on the sample such that enough photons can enter the sample to perform spectroscopy, and the detection region may comprise a large enough area on the sample to detect the scattered photons emerging from the sample.

However, if the illumination region is too large, there may be a large distribution in the scattered photon distances within the sample. It may be difficult to model the light scattered inside the sample, and thus it may be difficult to determine the composition of the sample. Thus, it may be useful to reduce the distribution of the path lengths travelled by the photons emerging at the detection region and detected by the detector, by reducing the area of the illumination pattern.

Thus, in some embodiments, the illumination pattern comprises a ring-shaped illumination pattern. For example, when the detector is arranged relative to the illumination module such that the detector field-of-view (i.e. the detection region) is placed in the centre of this ring, the distribution of distances between the detection region (e.g. the midpoint of the illumination pattern), and each point along the circumference of the illumination pattern is reduced, whilst maintaining a large illumination pattern to increase the signal-to-noise ratio. This may improve the detection of light that has been scattered deep into the sample.

In some embodiments, the processor is programmed to output the data representative of measurements from the detector and/or the image from the camera to an external processing unit, e.g. using a transmitter or wired connection. For example, the processor may be programmed to convert the measurements taken by the detector into a form that can be sent to the external processing unit. The external processing unit may be arranged to perform analysis on the measurements, in order to determine the composition of the sample. The processor may be programmed to first store the data before the data is sent to the external processing unit, e.g. the device may comprise a memory arranged to store the data from the detector, and/or other data used for processing the data.

The distance between the detection region and the illumination pattern may determine how deep the light penetrates before it emerges at the detection region and is detected by the detector. Thus, in some embodiments the processor or the external processing unit is programmed to model the path of light through the sample using the distance between the detection region and the illumination pattern and/or the position of the guiding pattern in the image captured by the camera, to determine the penetration of light into the sample.

In some embodiments, the processor or the external processing unit is programmed to adjust the path model depending on the relative distances of the illumination pattern and the guiding pattern and/or the position of the guiding pattern in the image captured by the camera. This can be used to more accurately determine the composition of the sample, e.g. when the light is modelled to have penetrated a given depth into the sample, the composition of the sample at that depth can be determined. This may allow for the composition of the sample to still be accurately determined when the device is not in the desired position relative to the sample (e.g. when the illumination pattern and the guiding pattern are not within the second predetermined distance). For example, the detected light may be indicative of the composition of the sample at a particular depth, e.g. closer to the surface.

In some embodiments, the processor is programmed to correct the data taken by the detector when the device moves during the measurement such that the guiding pattern and the illumination pattern are no longer within the second predetermined distance and/or the position of the guiding pattern in the image captured by the camera is not in the desired position. For example, the processor may indicate the depth at which the data has been taken based on the relative positions of the illumination pattern and the guiding pattern. This may further improve the usability of the device, as the user may not need to perfectly keep the device in the same place during the measurement in order to obtain an accurate measurement of the sample composition.

The guiding module is arranged to project the guiding pattern in the second direction at an angle relative to first direction in which the illumination module projects the illumination pattern, such that the position of the guiding pattern may be in the predetermined position in the image and/or the relative positions of the guiding pattern and the illumination pattern on the sample may be within the second predetermined range when the device is positioned between 5 and 20 cm from the sample, e.g. approximately 10 cm. The spacing between the device and the sample at which the guiding pattern is in the predetermined position in the image and/or the guiding pattern and the illumination pattern are within the second predetermined range may depend on the arrangement of the guiding module, illumination module and the detector within the device, which may be selected based on the sample to be analysed.

The working distance (i.e. the distance between the device and the sample when the field-of-view of the detector and the illumination pattern are within a first predetermined range) may be changed depending on the sample being imaged, as it may be set by the geometry of the detector, the illumination module, and the guiding module within the device, e.g. the relative angles of the field-of-view of the detector, and the projection of the guiding pattern and the illumination pattern. For example, a different geometry (e.g. respective angles) of detector, illumination module and guiding module may be needed to provide different working distances, e.g. for taking measurements of different areas of samples, or in different background light conditions.

In some embodiments, the detector comprises a spectrometer, wherein the detector is arranged to take measurements of the wavelengths and intensities of light scattered inside the sample. In some embodiments, the spectrometer is arranged to perform visible to near-infrared interactance spectroscopy (VIS/NIRS), e.g. the detector is arranged to detect light that has been scattered after interacting with the sample within a wavelength range of 400 - 1200 nm, e.g. 700 - 1100 nm. For example, the measurements taken by the detector may comprise a spectrum of the wavelength and intensity of the light scattered inside the sample.

VIS/NIRS is a technique that can be used for determining the amount of fat, water, protein, and other substances in food, by measuring molecular vibrations involving hydrogen bonds. The device may therefore be used for rapid and non-destructive determination of food quality. For example, the device may be used to determine the meat content in the legs of a (e.g. king) crab. The legs of crabs contain muscle tissue and haemolymph (containing water), and thus the amount of meat within a crab leg can be assessed by measuring the ratio of protein and water using VIS/NIRS. A higher ratio of protein to water is indicative of a higher meat content, and hence a higher quality crab leg.

In some embodiments, the processor or the external processing unit is programmed to determine the composition of the sample based on the data (e.g. VIS/NIRS spectra) measured by the detector and output an indication of the composition of the sample, e.g. one or more parameters of the sample. For example, the indication may comprise an indication of the bulk composition of selected substances in the sample, e.g. the percentage of fat in the sample.

The processor or the external processing unit may apply a predetermined algorithm to the VIS/NIRS spectra obtained by the detector, to determine the composition of the sample, e.g. to determine the percentage of one or more different substances in the sample such as protein, water, sugar, and/or other substances. The algorithm may use a predetermined model that is adapted to work on data recorded with the desired geometry between the illumination pattern and the detection region.

For example, the processor or the external processing unit may be programmed with an algorithm which uses the predetermined model, where the predetermined model is created using a database of previously recorded spectra of known sample compositions. The algorithm may adjust various parameters of the model until the model substantially matches the measured spectrum, and the processor may be programmed to output the sample composition based on the adjusted parameters of the refined model. The model may be stored in a memory connected to the processor, e.g. onboard the device or externally. Thus, the device may only store the model, and not any previously recorded spectra (e.g. in a memory), to determine the sample composition.

The relative intensities of different wavelengths of light may be used by the processor to determine the relative quantities of these different substances, in order to determine the composition of the sample. For example, the processor may be programmed to output the indication of the composition of the sample via a user interface. The parameters of the model may be initially selected by the processor or the external processing unit based on the distance between the illumination pattern and the detection region during the measurements taken by the detector, e.g. based on the model of the path of light taken through the sample.

Preferably, the detector is arranged to detect light within a wavelength range of 700-1100 nm. The light scattered inside the sample within this range may be light that has interacted the most with the sample, e.g. the light that has penetrated farthest below the surface of the sample. Thus, it may be useful to detect light in this range, in order to determine the composition of the sample deeper below the surface, e.g. 5-15 mm below the surface of the sample. Conventional detectors often have poor sensitivity at wavelength ranges above 1000 nm, and almost zero sensitivity above 1100 nm as this range is at the boundary of the wavelength detection ranges of typical Si detectors. Detectors such as InGaAs detectors may be needed to measure longer wavelengths (e.g. above 1000nm), which may be more expensive.

In some embodiments, the illumination module is arranged to project broadband (e.g. white) light. Preferably, the illumination module is arranged to project light within the wavelength range that may be detected by the detector, e.g. between 400 and 1100 nm. For example, light within this wavelength range may be useful for characterising the sample, as these wavelengths of light may interact with the sample more than other wavelengths. Preferably, the first light source is arranged to emit light in the visible range of the electromagnetic spectrum. This may allow for the illumination pattern may to be visible to the user and therefore be used for aligning the illumination pattern and the guiding pattern. For example, the first light source may comprise a halogen bulb, which may provide a more reliable and stable spectrum compared to a light emitting diode (LED).

In some embodiments, the illumination module comprises a mask, wherein the mask is arranged to partially block the light projected by the first light source. This may enable the illumination module to project differently shaped projection patterns, depending on the shape of the mask used.

In some embodiments, the illumination module is arranged to project the illumination pattern to surround the field-of-view of the detector (i.e. to surround the detection region). For example, the illumination pattern may comprise two lines on either side of the detection region (e.g. when the illumination pattern and the guiding patterns are within the second predetermined range). Preferably, the illumination pattern comprises at least a portion of a ring, wherein the detection region is in the centre of the ring. This may help to maximise the light that is able to interact below the surface of the sample and be scattered (and thus detected) at the detection region, as the light is able to reach the detection region from all sides of the sample. For example, the illumination module may comprise a doughnut-shaped mask arranged to produce the ring-shaped illumination pattern.

The distance between the detection region and the illumination pattern may be selected to help optimise the interactance between the light and the sample, in order for the measurements taken by the detector to be used to determine various properties of the sample. For example, the distance between two sides of the illumination pattern may be between 8 mm and 30 mm cm, with the detection region centred between the two sides of the illumination pattern.

The use of a mask over the first light source in order to project a particular illumination pattern may allow the projected illumination pattern to be a sharp light pattern, e.g. the pattern is well defined. This may enable the distance light travels through the sample before it is scattered and re-emerges at the detection region to be well known, and thus the spectra produced to be well defined, e.g. to make it easier to determine the composition of the sample from the spectra.

In some embodiments, the guiding module is arranged to project the guiding pattern onto the field-of-view of the detector (i.e. onto the detection region) e.g. the guiding pattern indicates the field-of-view of the detector. Preferably, the second light source is arranged to emit light in the visible range of the electromagnetic spectrum. This may help the guiding pattern to be visible to the user of the device, e.g. to allow for the user to see which region of the sample they are detecting. For example, if the sample is heterogenous, it may be useful for the user to know which part of the sample they are analysing, e.g. looking at the leg vs the body of a crab. This may further improve the accuracy when imaging a particularly small sample, e.g. a strawberry.

In embodiments where the device comprises a camera, the camera may be arranged to image infra-red light, e.g. to image the illumination pattern. Preferably, the camera is arranged to image visible light (e.g. in addition to imaging infra-red light), e.g. to image the guiding pattern (e.g. and the illumination pattern).

In embodiments where the detector is used for VIS/NIR spectroscopy, it may be useful for the second light source to emit visible light at a wavelength outside of the detection range of the detector, such that the guiding pattern is less likely to affect the measurements and does not need to be turned off during operation of the detector.

In embodiments comprising a camera, the processor is programmed to determine the number of pixels between the guiding pattern and the illumination pattern in the image captured by the camera (e.g. when in the guiding mode), wherein the second predetermined range is a range of pixels. For example, the second predetermined range may be when the edge of the guiding pattern is within a particular number of pixels of the relative to the edge of the illumination pattern, e.g. when the circular-shaped guiding pattern is within approximately 5 pixels of the centre of the ring-shaped illumination pattern. The visibility of the guiding pattern may be improved by using a filter on the camera to suppress wavelengths outside the range of the wavelength(s) projected by the guiding module.

In some embodiments, the processor is programmed to operate the detector to take a measurement for between approximately 0.5 s - 1 s. This may be the time taken for the detector to obtain a spectra of the light received from the detection region of the sample once the detector is turned on. For example, this may be useful when the sample is a living creature e.g. a crab, which may move whilst the measurement is being taken, and thus it could be difficult to keep the device in the desired position (e.g. at a 'correct' distance such that the field-of-view of the detector and the illumination pattern are within a first predetermined range) relative to the crab. Preferably, the measurement is repeated at least twice in order to obtain a high signal to noise ratio.

In some embodiments, the processor is programmed to turn off the guiding module, e.g. the processor is programmed to turn off the second light source when the detector is taking measurements (e.g. in the illumination mode). Preferably, the guiding module is arranged to project the guiding pattern and the processor is programmed to compare the position of the guiding pattern in the image and/or the relative positions of the guiding pattern and the illumination pattern during the measurements (e.g. in illumination mode). For example, the processor may be programmed to run the illumination mode and guiding mode concurrently, such that the guiding pattern and illumination pattern are projected at the same time. This may be useful to help ensure that the device is still in the desired position relative to the sample whilst the measurements are being taken by the detector.

Preferably, the detector and/or processor is arranged (e.g. programmed) to filter out the guiding pattern. For example the processor may be programmed to filter out the wavelengths of light corresponding to the guiding pattern from the measurements output or stored, and/or the device (e.g. the detector) comprises a filter arranged to filter out the wavelengths of light from the guiding pattern from reaching the detector. This may prevent light from the guiding module affecting the measurements taken by the detector, whilst enabling the position of (e.g. distance between) the device and the sample to be checked.

In some embodiments where the device comprises a camera, the device comprises a user interface connected to the processor, wherein the user interface is arranged to indicate to the user (e.g. a person or a robot) when the position of the guiding pattern in the image is in the predetermined position and/or the guiding pattern and illumination pattern are within the second predetermined range based on the image captured by the camera (e.g. when the device is in the desired position relative to the sample to take measurements). The user would then know to keep the device in that particular position, whilst measurements are taken by the detector.

In embodiments where the user visually determines whether the device is in the desired position (e.g. at the 'correct' distance) and the device comprises a camera, the user interface is preferably arranged to feedback to the user to move the device relative to the sample when the illumination pattern and guiding pattern are not within the second predetermined range, and/or when the position of the guiding pattern is not in the desired position in the image. For example, the user interface may comprise a screen arranged to output visual feedback, speaker arranged to output aural feedback, and/or vibrator arranged to output haptic feedback.

In some embodiments, the user interface is arranged to provide continuous feedback of the position of the device relative to the sample when the detector is taking measurements, e.g. during the illumination mode. This may ensure that the device is held in the right place relative to the sample throughout the measurement, such that the measurements taken by the detector may be more accurate. For example, the speaker may be arranged to output a noise, e.g. a sequence of 'beeps', depending on the distance of the device from the sample. The screen may be arranged to indicate to the user to move the device forwards, backwards, or to tilt the device, e.g. via a corresponding action displayed on the screen. The vibrator may be arranged to vibrate, e.g. change the frequency of vibrations, depending on the distance of the device from the sample.

In some embodiments, the device is arranged to be mounted to a robot, e.g. the processor is programmed to be connected to a processing unit of a robot. For example, the device may be arranged to be mountable onto an arm of the robot. The processor may be programmed to output a signal to the robot, indicating that the device needs to be moved relative to the sample, based on the relative distances of the guiding pattern and illumination pattern. This may enable a robot to take automated measurements of various samples.

In some embodiments, the device is arranged to measure the background light (e.g. ambient light), and the processor is programmed to output or store data representative of the measurements of background light from the detector. For example, the processor may be programmed to, before the detector is turned on to take measurements, turn off the illumination module (e.g. the first light source) whilst the detector is turned on, in order to take a measurement of the background light. The device may be arranged to then turn the illumination module back on, in order to take measurements of the light scattered inside the sample. The processor may be programmed to store or output the data representative of the measurements background light taken by the detector, e.g. by subtracting the background light from the data representative of the light scattered from the detection region taken in the illumination mode. Subtracting the background light may also correct for any potential offset in the signal received at the processor from the detector.

In some embodiments, the illumination module comprises a shutter, wherein the shutter is arranged to periodically block the light from the first light source (e.g. block the light from the first light source from reaching the sample). For example, the shutter may comprise a wheel comprising a window rotating at a fixed frequency. The shutter may be arranged to toggle between open and closed (i.e. between the illumination module being 'on' and 'off').

This may allow for correction of the background light received at the detector, and thus the detected NIR spectra, e.g. without needing to turn the first light source on and off. For example, the processor may be programmed to subtract the measurement of background light from the measurement of light from the first light source scattered inside the sample. This may also correct any offset bias in the detector.

The user interface may be arranged to indicate when the background light is above a particular threshold, as the detector may not be able to accurately measure the light scattered inside the sample when there is too much ambient light. When the background light is very high, or very variable, the detected spectra may be of poor quality, and the data may be discarded by the processor.

In some embodiments, the processor is programmed to operate the shutter when operating the detector to take measurements, such that the detector is arranged to measure the background light when the shutter is closed and measure the light scattered inside the sample when the shutter is open; and

wherein the processor is programmed to output or store data representative of the measurements of background light from the detector. For example, for each toggle period of the shutter, the quality of the data may be determined by comparing the variation in background light between the toggle periods.

By correcting for the background light during the measurement period using the shutter (e.g. wherein the shutter is operated at a particular frequency), the device may be able to correct for variations in the position of the device, and for variations in background light during the measurement period. The required shutter frequency may be dependent on the variation in the background light during the measurement. For example, as the measurement is non-contact and the device is portable, e.g. handheld, the sample and/or device is likely to move slightly during the measurement period. This relative motion may cause a change in the background light, which may be corrected for by the processor using the measurements received when the shutter is closed. For example, when the background light is substantially constant, the shutter frequency may be approximately 1 Hz. Furthermore, when the device is used outside, the background light may be variable, e.g. due to cloud cover, and thus the device may be able to correct for this using the shutter arrangement and be used outside. For example, when the background light is variable, a higher shutter frequency of 5 - 50 Hz may be used. This is useful for in-situ measurements of food produce, which typically would have to be measured in lab conditions using presently known detection methods. This may also be useful when determining the stability of the measurements by comparing the intensity of measurements over time, in an environment with variable background light.

In some embodiments the processor is programmed to determine the quality of the measurement taken by the detector, and output or store the data representative of the quality of the measurement. For example, the processor may be programmed to receive the measurements of the background light, the stability of the device or sample during the measurement, and/or compare the measurements of wavelength/intensity taken by the detector to expected spectra for the sample being measured.

This may allow the processor to determine whether the spectrum taken by the detector is of a high enough quality to accurately determine the composition of the sample. For example, if the spectrum output by the detector was significantly different to that which was expected (e.g. the measured light intensity is higher than expected), the detector may be detecting the light reflected from the surface of the sample rather than the light that has been scattered inside (i.e. that has penetrated into and interacted inside) the sample, and so it is likely that the measurement is of poor quality and should be taken again. This may be used to determine whether the device is working as expected.

In embodiments where the device comprises a user interface, the user interface may be arranged to indicate when the quality of the measurement taken is below a particular threshold. For example, the user interface may be arranged to indicate that the measurement needs to be taken again, e.g. because the device was moved too much during the measurement.

In some embodiments, the illumination pattern is selected based on the sample being measured, e.g. based on the size of the sample. For example, if the sample is small, e.g. a strawberry, the illumination pattern may be small, when compared to a larger sample e.g. a crab. For example, the larger the illumination pattern projected onto the sample, the greater the range of potential photon paths, and thus the greater the range of potential photon path lengths. This may allow the detector to detect light that has interacted deeper into the sample, and/or may increase the signal to noise ratio of the detector. However, if the illumination pattern projected onto the sample is too large, it may miss parts of the sample, e.g. a strawberry not receive any light from an illumination pattern that is too large. Furthermore, using a small illumination pattern for a large sample may give a measurement at the detector that is not representative of the sample, e.g. the light received at the detector may have only travelled through a very small part of the sample that is not representative of the sample as a whole (e.g. a blemish), and thus give an incorrect determination of the composition of the sample.

In some embodiments, the illumination module is arranged to project at least two different illumination patterns, and each illumination pattern is independently projectable. For example, the illumination module may comprise two different light sources, which may be arranged to be turned on and off independently. The illumination module may comprise one or more masks, wherein the masks are arranged to be changed depending on the illumination pattern to be projected.

For example, a first illumination pattern may be used for larger or more opaque objects (e.g. crabs), and a second illumination pattern may be used for smaller or more translucent objects (e.g. strawberries). The device may be arranged to switch between the first and second illumination patterns, e.g. when the user selects the size of the sample being measured via the user interface.

The illumination module may be arranged such that the same guiding pattern and detector may be used regardless of which illumination pattern is being projected. For example, the second predetermined range between each illumination pattern and the guiding pattern may be fixed, however the device may need to be a different distance from the sample for different illumination patterns, in order for the patterns to be aligned. For example, the first and second illumination patterns may each comprise one half of a ring, wherein the first illumination pattern may project a larger area onto the sample than the second illumination pattern.

Features of any aspect or embodiment described herein may, wherever appropriate, be applied to any other aspect or embodiment described herein. Where reference is made to different embodiments or sets of embodiments, it should be understood that these are not necessarily distinct but may overlap.

Certain embodiments of the invention will now be described, by way of example only, with reference to the accompanying drawings, in which:
Figure 1 is a schematic diagram of device for determining the composition of a sample;
Figure 2 is a schematic diagram of the light projected onto the sample by the device shown in Figure 1;
Figures 3a-3c are a schematic diagram of the relative positions of the first illumination pattern and the guiding pattern;
Figure 4a is a schematic diagram of the illumination module shown in Figure 1;
Figure 4b is a view of the mask shown in Figure 4a;
Figure 4c is a schematic diagram of an alternative illumination module to that shown in Figure 4a;
Figure 5 shows an example of the intensity of the signal detected at the detection module with respect to time and the operation of the shutter in the illumination module;
Figures 6a and 6b are schematic diagrams of a first and second illumination pattern respectively;
Figure 7 is a schematic diagram of the detection module shown in Figure 1;
Figure 8 is a block diagram of the flow of information in the device shown in Figure 1;
Figure 9 is a flow diagram of the method carried out by the device shown in Figure 1;
Figure 10 shows near-infrared interactance spectra taken by the device shown in Figure 1.

Various devices will now be described in the context of performing near-infrared interactance spectroscopy for determining the composition of a food sample.

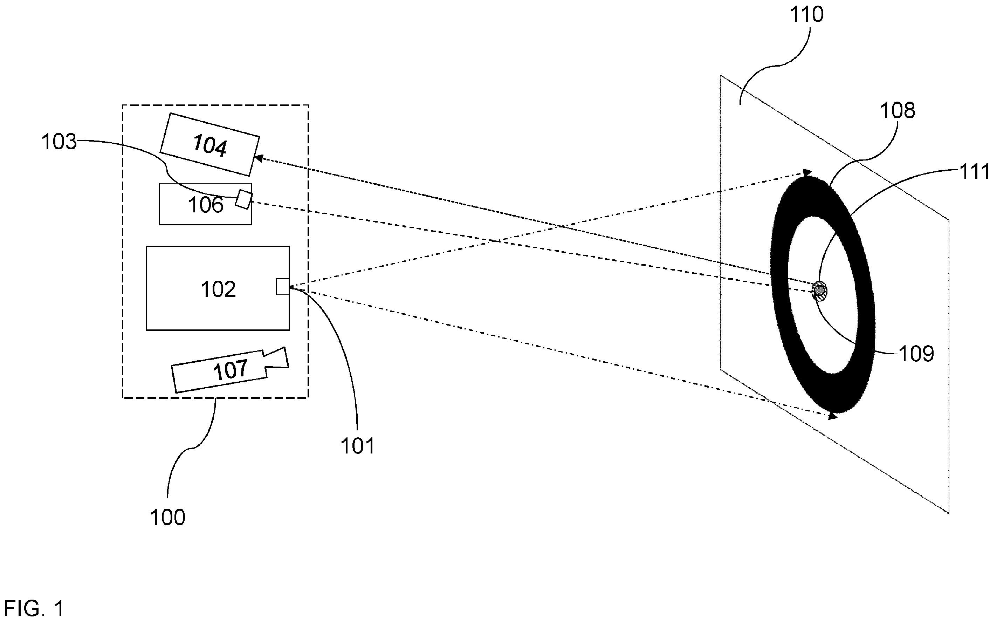

Figure 1 shows a schematic diagram of a device 100 for determining the composition of a sample 110. The device 100 includes an illumination module 102, a guiding module 106, a detector 104, and a camera 107.

The illumination module 102 includes a first light source 101, and projects an illumination pattern 108 onto the sample 110, where the photons from the illumination pattern 108 are either transmitted, absorbed, scattered or reflected by the sample 110. Depending on the type of sample 110 (e.g. the composition of the sample 110), different wavelengths light will be transmitted, absorbed, and scattered/reflected by the sample. The detector 104 is a spectrometer that measures the wavelength and intensity of light that from the illumination pattern 108 that is internally scattered inside the sample 110, in order to produce a spectrum of the scattered light.

The detector 104 is positioned to receive scattered light from a detection region 111 on the surface of the sample 110 that is spaced from the location of the illumination pattern 108 projected onto the sample. The light that is scattered from the region spaced from the illumination pattern 108 is likely to be light that has interacted with the sample, e.g. light that partially penetrated the sample before it is scattered. The detector can therefore probe the interior of the sample to the depth reached by the light from the illumination pattern 108 before being scattered and re-emerging at the detection region 111. The detector 104 performs near-infrared interactance spectroscopy (NIRS) on the sample 110, which may be used determine the composition of the sample from the scattered light.

The measurements from the detector 104 are processed by a processor onboard the device 100, or they are sent to an external unit (not shown) to be processed, in order to determine the composition of the sample. The spectrum measured by the detector 104 is compared to other spectra, or the peaks of the spectrum are individually analysed, to identify particular chemical signatures (e.g. from protein or water) and determine the composition of the sample.

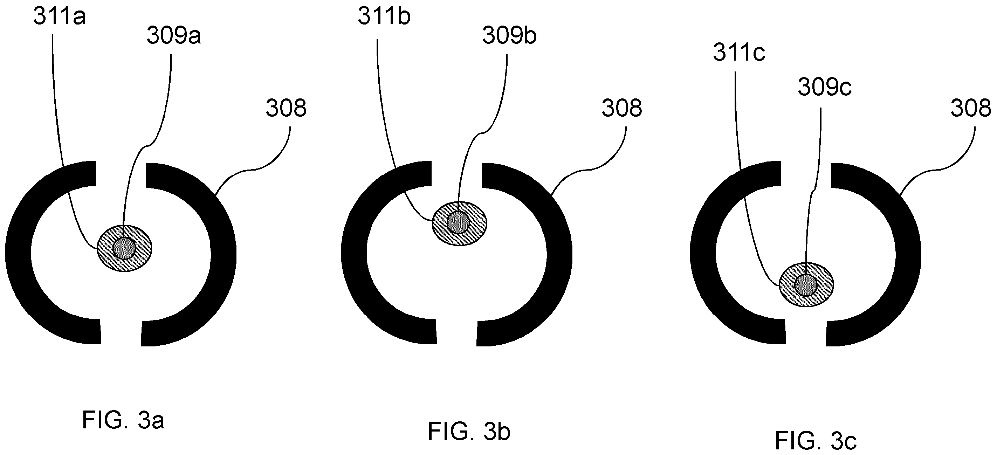

The guiding module 106 includes a second light source 103 and projects a guiding pattern 109 onto the sample. The relative positions of the guiding pattern 109 and the illumination pattern 108 on the surface of the sample are used to determine whether the device 100 is in the desired position (e.g. distance) relative to the sample. The guiding module 106 projects light from the second light source 103 at an angle relative to the light projected by the illumination module 102 from the first light source 101. This means that the relative positions of the guiding pattern and the illumination pattern on the sample vary depending on the position of the device relative to the sample, as described with reference to Figures 3a-3c.

When the guiding pattern 109 and the illumination pattern 108 are within a predetermined range from each other on the surface of the sample (e.g. within a predetermined number of pixels in an image captured by the camera 107), the detector 104 is in the correct position to be able to take measurements of the light that is scattered inside the sample (e.g. after penetrating the sample).

The guiding pattern 109 on the sample indicates the field-of-view (i.e. the detection region 111) of the detector 104. The guiding pattern 109 being within the predetermined range from the illumination pattern 108 means that the field-of-view of the detector 104 is adjacent to and spaced from the illumination pattern, and the detector 104 can perform NIRS.

The second light source 103 emits visible light, e.g. green light. This allows the user of the device 100 to see which part of the sample they are aiming the device at, i.e. which part of the sample they are measuring the composition, and may help the user to see if the illumination pattern is in the desired position relative to the guiding pattern (e.g. where the illumination pattern includes a visible component).

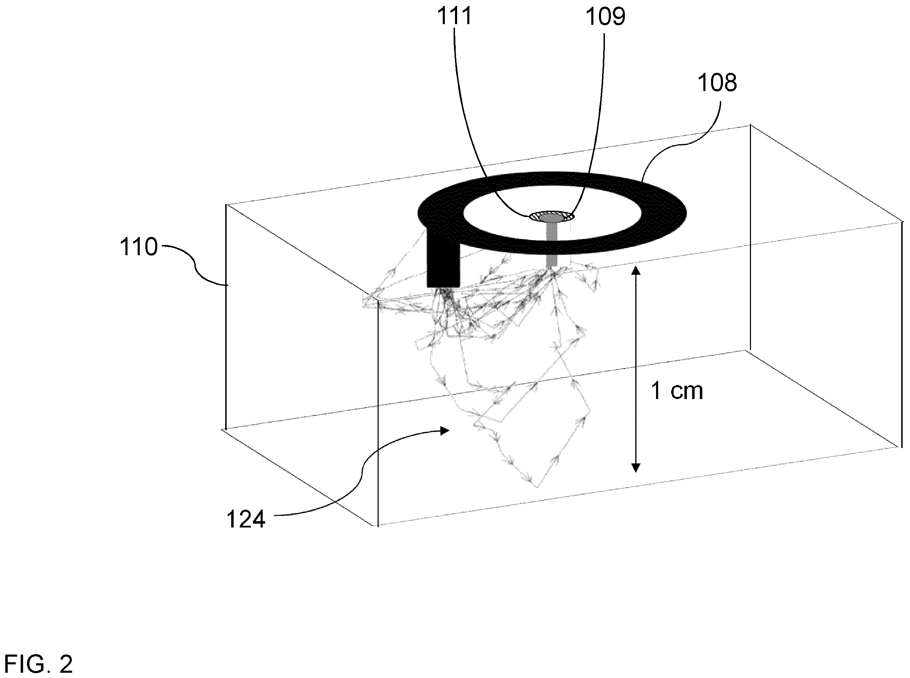

As shown in Figure 1, the illumination pattern 108 is a ring, and the guiding pattern 109 is a dot. When the device 100 is at the desired distance from the sample 110, the guiding pattern 109 dot is approximately in the centre of the illumination pattern 108 ring. This arrangement may maximise the amount of light from the illumination module 102 that has penetrated deep into the sample, and has been scattered such that it is detected by the detector 104 within the detection region 111 at the position of the guiding pattern 109. By spacing the illumination pattern 108 from the guiding pattern 109, the detector 104 is able to detect the photons from the illumination pattern 108 that have been internally scattered deep within the sample and reached the position of the guiding pattern 109, i.e. the field-of-view 111 of the detector 104, as described with reference to Figure 2. Furthermore, by spacing the illumination pattern 108 from the guiding pattern 109, the detector 104 detects fewer photons that have only weakly penetrated the sample (e.g. that have only been internally scattered near the surface of the sample) and thus do not contain as much information about the composition of the sample deep below the surface. This may improve the quality of the determination of the composition of the sample below the surface using the measurements taken by the detector 104.

The first light source 101 emits light in a wavelength range of 400 nm - 1100 nm. Preferably this includes a component in the visual spectrum, which allows the user to visually align the illumination pattern 108 and the guiding pattern 109. The wavelength range is also selected for characterising the sample using VIS/NIRS.

The camera 107 is positioned to image the surface of the sample 110, including the illumination pattern 108 and the guiding pattern 109 projected onto the surface of the sample. The camera 107 is thus able to detect the wavelength ranges of both the first light source and the second light source. The camera captures an image (or images) of the relative positions of the illumination pattern 108 and the guiding pattern 109, which is sent to a processor to determine whether the device is in the desired position relative to the sample.

The relative distance between the guiding pattern 109 and the illumination pattern 108 is used by the device and the user to determine whether the device is in the desired position relative to the sample for the detector 104 to take measurements for VIS/NIRS. If the device is in the desired position, the processor (or the user) instructs the detector 104 to take measurements of the scattered light.

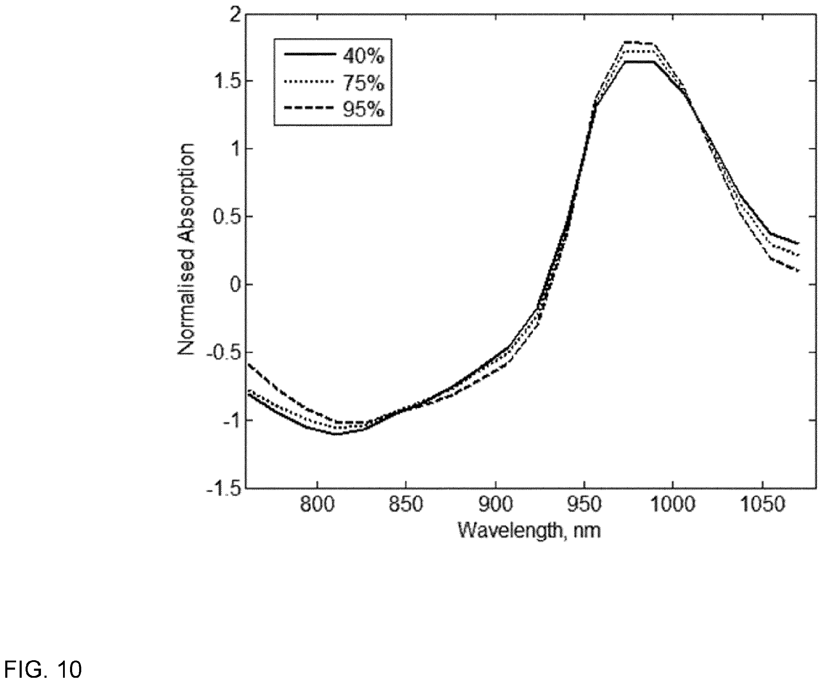

The sample 110 could be any substance, but preferably it is a foodstuff containing water. The detector 104 is able to perform VIS/NIRS to determine the percentage of water and other substances present in the sample. For example, where the sample is a crab leg, the device 100 can be used to determine the percentage of the crab leg that is water, and the percentage that is protein (i.e. meat). An example VIS/NIRS spectrum is described with reference to Figure 10.

Figure 2 shows schematically the light from the illumination module 102 interacting with the sample 110. The light from the first light source 101 enters the sample 110 at the surface of the sample where the detection region 108 is projected. The light (formed of multiple photons 124) that enters the sample is scattered, reflected, absorbed, and transmitted by the sample 110. Some of the photons 124 are internally scattered (e.g. multiple times) inside the sample 110, until they are scattered out of the sample 110. The photons 124 that are scattered out of the sample at the point where the guiding pattern 109 is projected onto the sample (i.e. the field-of-view of the detector 104) are detected and measured by the detector 104. The photons 124 penetrate a few centimetres (e.g. 1 cm) into the sample, and thus the measurements taken by the detector 104 are representative of the composition of the sample down to a few centimetres below the surface.

The guiding module 106 is positioned to project the guiding pattern 109 at an angle relative to the angle of the illumination pattern 108. This means that that the illumination pattern 108 and the guiding pattern 109 will only be aligned when the device 100 is held at a particular distance relative to the sample 110.

Figures 3a-3c show how the relative positions of the illumination pattern and the guiding pattern vary depending of the position of the device 100 relative to the sample 110. Figure 3a shows the relative positions of an illumination pattern 308 and the guiding pattern 309a when the device is in the desired position relative to the sample, with the detection region 311a surrounding the guiding pattern 309a. The guiding pattern 309a and illumination pattern 308 are visible to the user, however the detection region 311a is not, and is for illustrative purposes only. Figure 3b shows the relative positions of the illumination pattern 308 and the guiding pattern 309b when the device is too far away from the sample, with the detection region 311b surrounding the guiding pattern 309b. Figure 3c shows the relative positions of the illumination pattern 308 and the guiding pattern 309c when the device is too close to the sample, with the detection region 311c surrounding the guiding pattern 309c.

It will be appreciated that if the detector and guiding module were positioned below the illumination module in the device, the relative positions of the illumination pattern 308 and guiding pattern 309b in Figure 3b would be indicative of the device being too close to the sample, and the relative positions of the illumination pattern 308 and guiding pattern 309c in Figure 3c would be indicative of the device being too far from the sample. This means that the correspondence between the relative positions of the guiding and illumination patterns and the distance of the device from the sample are dependent on the device geometry, and Figures 3a-3c and are only representative of the arrangement in Figure 1.

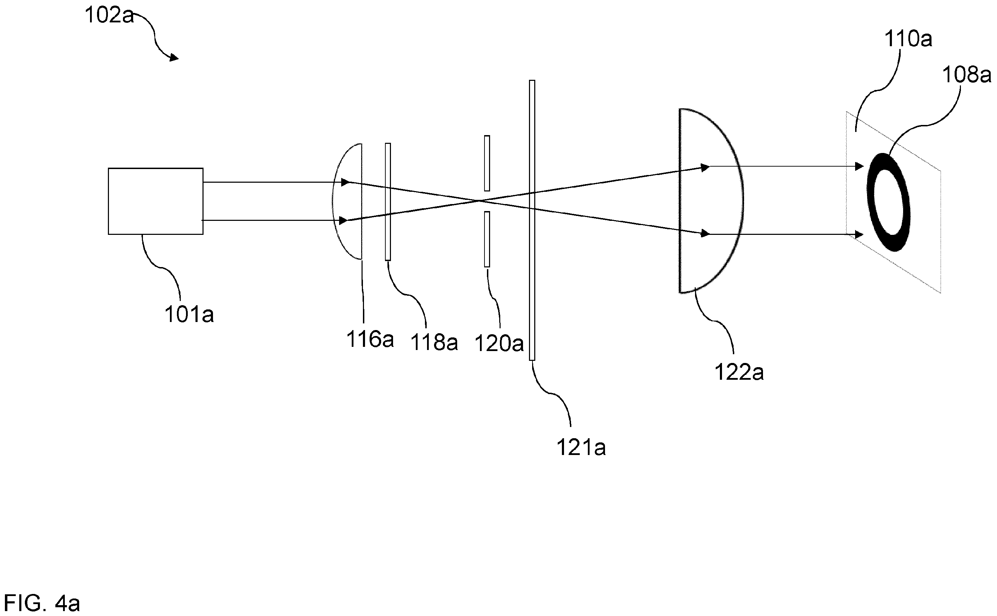

Figure 4a shows a schematic diagram of the illumination module 102a shown in Figure 1. The illumination module 102a includes the light source 101a (e.g. a halogen lamp), a lens 116a, a mask 118a, an aperture 120a and shutter 121a, and an imaging lens 122a. The illumination pattern 108a produced by the illumination module 102a is projected onto the sample 110a.

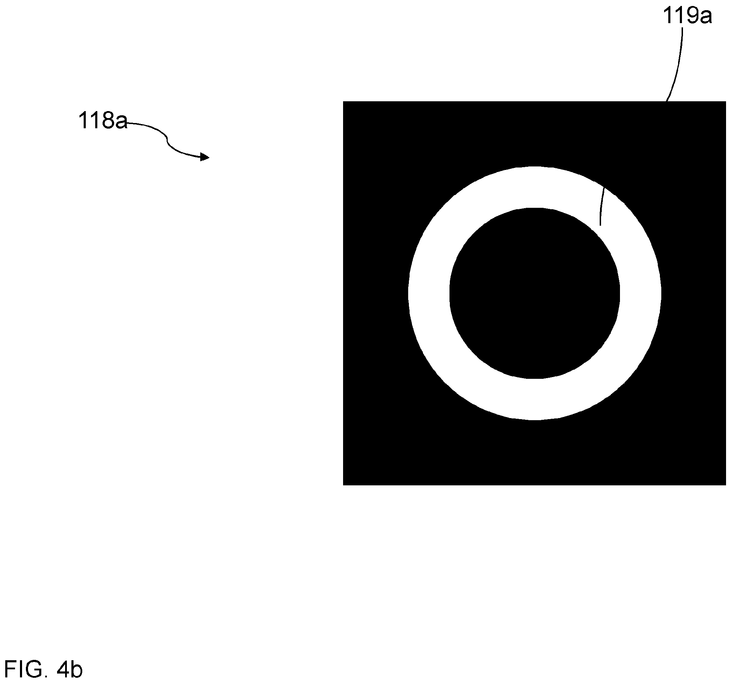

The light source 102a shines light (e.g. white light) onto the lens 116a, which in turn focuses the light onto the mask 118a. The mask 118a is shown in more detail in Figure 4b, and is an opaque mask which only allows light from the halogen lamp to pass through the ring-shaped hole 119a, such that the illumination module 102a projects the ring-shaped illumination pattern 118a.

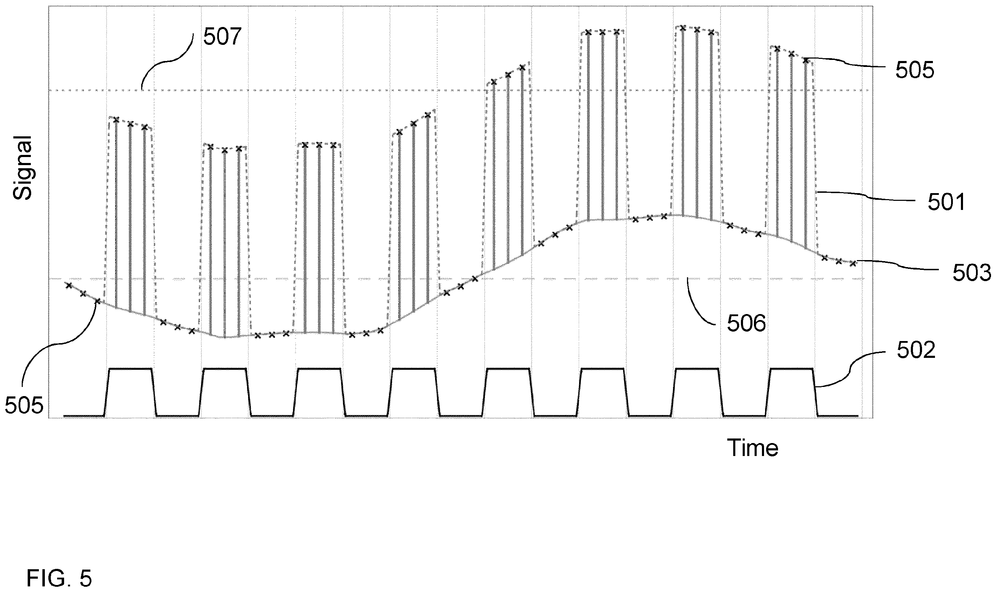

The ring-shaped light pattern emerging from the mask 118a passes through the aperture 120a (i.e. when the shutter 121a is open). The shutter 121a is controlled by the processor to open and close in order for the detector to take measurements of the background light when the shutter 121a is closed, and of the light scattered inside the sample 110a when the shutter is open. The operation of the shutter is described in more detail with reference to Figure 5.

The light then passes through the imaging lens 122a, which focuses the ring-shaped light pattern onto the surface 110a of the sample, thus projecting the ring-shaped illumination pattern 118a.

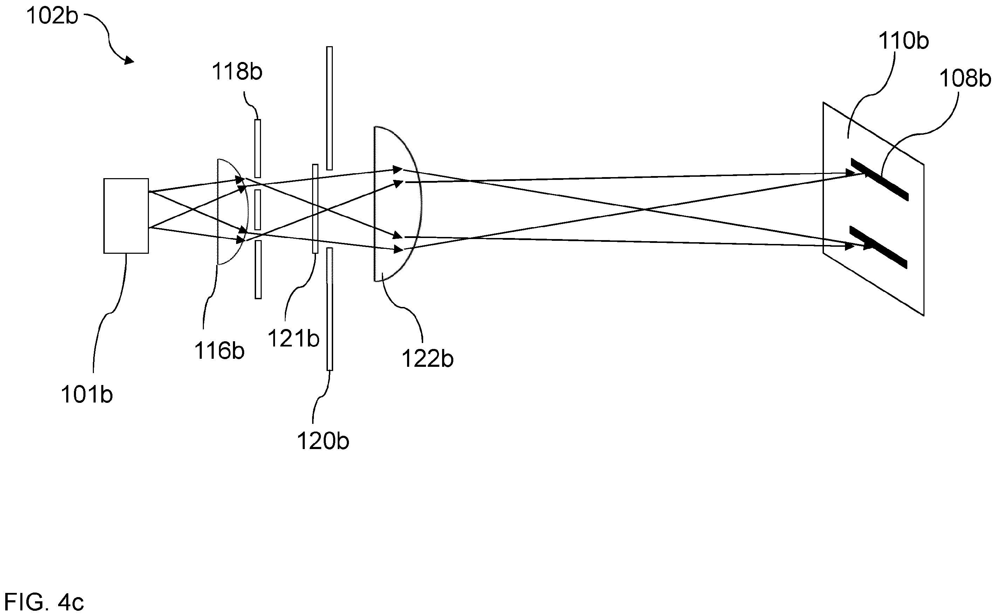

Figure 4c shows an alternative illumination module 102b to the illumination module shown in Figure 4a, and includes a light source 101b, a lens 11b, a mask 118b, an aperture 120b, a shutter 121b, and an imaging lens 121b. The illumination pattern 108b produced by the illumination module 102b is projected onto the sample 110b.

The light source 101b shines light (e.g. white light) onto the lens 116b, which in turn focuses the light onto the mask 118b. The lens 116b may be the same lens, or different to the lens shown in Figure 4a, e.g. it may include a parabolic mirror. The mask 118b is an opaque mask which only allows light from the light source 101b to pass through two slits, such that the illumination module 102b projects the double slit illumination pattern 118b.

The double slit illumination pattern emerging from the mask 118b passes through the shutter 121b when it is open, and through the aperture 120b. The shutter 121b is before the aperture 120b, in comparison to the illumination module 102a where the shutter 121a is after the aperture 120a. The shutter 121b is controlled by the processor to open and close in order for the detector to take measurements of the background light when the shutter 121b is closed, and of the light scattered inside the sample 110b when the shutter is open.

The light then passes through the imaging lens 122b, which focuses the double slit light pattern onto the surface 110b of the sample, thus projecting the ring-shaped illumination pattern 118b.

Figure 5 shows an example diagram of the intensity of light detected at the detector with respect to time, compared to the opening and closing of the shutter. For this example measurement, the shutter is opened and closed 8 times for a single NIRS measurement, to correct for background light.

A shutter line 502 shows when the shutter is open (high signal) and closed (low signal). The overall signal intensity detected at the detector is shown by the signal dotted line 501, with individual measurements taken indicated by the crosses 505.

When the shutter is closed, the detector takes measurements of the background light intensity, shown by the solid background line 503. The average background light intensity is shown by the dotted background line 506. When the shutter is open, the detector takes measurements of the total light intensity, including the background light and the light from the first light source scattered inside the sample and emerging from the detection region, shown by the by the total signal dotted line 501. The average total light intensity is shown by the dotted total line 507. In order to obtain the background corrected signal, the average background light intensity 506 is subtracted from the average total light intensity 507, to obtain an average of the light from the first light source scattered inside the sample. The spectrum obtained from the measurement process of Figure 5 will thus be corrected for background light.

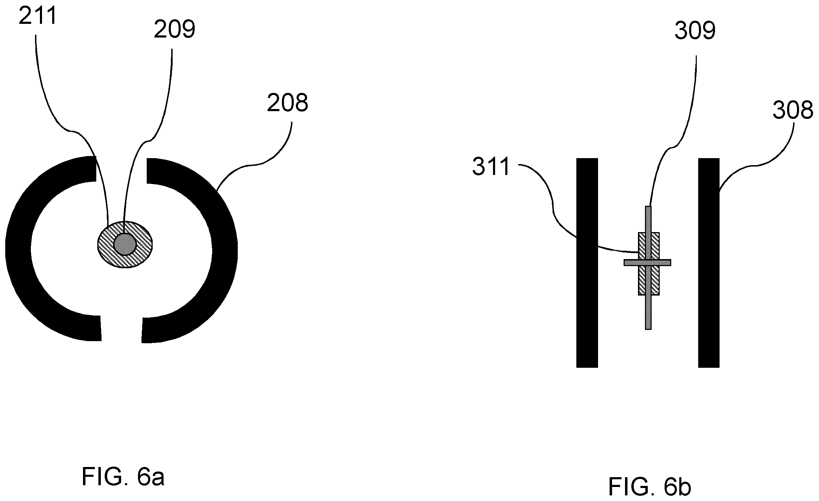

Figure 6a and 6b each show a different illumination pattern and guiding pattern that could be projected onto the sample 110. The illumination pattern is selected based on the size and opacity of the object being measured, and also the desired scattering/probing depth. For example, some samples may be heterogenous at a particular depth, and thus NIRS may be ideally performed at this depth, e.g. 5 mm. Thus, illumination pattern may be chosen to be closer, or further away from the detection region 111, to probe shallower or deeper respectively, based on the sample. It will be appreciated by the skilled person that other illumination patterns to what are shown are possible, e.g. a single straight line. The guiding pattern is chosen based on the Illumination pattern, to make it easier for the user or processor to determine when the guiding pattern and illumination pattern are within the predetermined range.

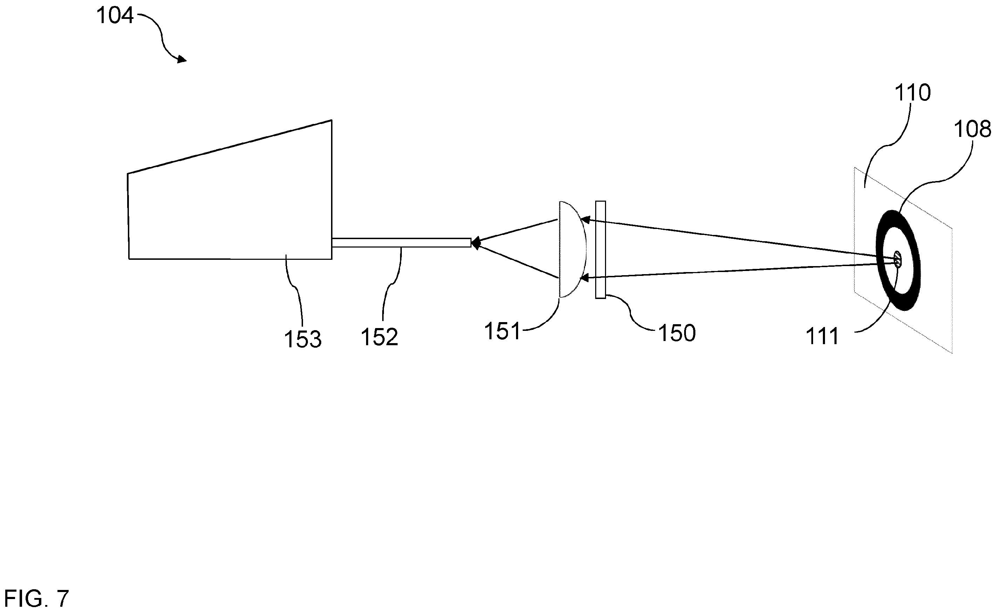

Figure 7 shows a schematic diagram of the detector 104 shown in Figure 1. The light from the first light source/illumination pattern 108 that interacts with and is scattered inside the sample 110 emerges at the detection region 111. The detector 104 is focused onto the detection region 111, to receive the light emerging from this region. The scattered light passes through a filter 150 for blocking any wavelengths that are not useful for measuring VIS/NIRS spectra of the sample, e.g. the wavelength of the guiding pattern.

The scattered light then passes through a detector lens 151, to focus the scattered light into a light homogeniser 152, which also includes a mixer and a waveguide. This smooths and focuses the scattered light into a spectrometer 153, which analyses wavelengths and intensities of the scattered light, to produce a VIS/NIRS spectrum. The obtained spectrum is then sent to the processor, where it is corrected for background light, and eventually analysed to determine the composition of the sample 110.

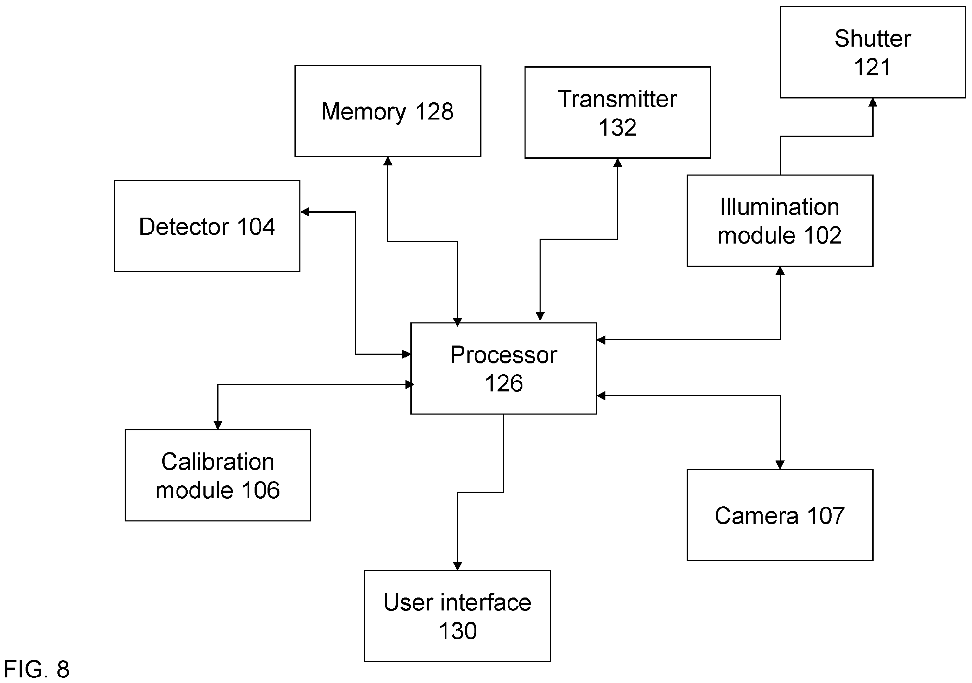

Figure 8 shows schematically a control system for operating the device 100 shown in Figure 1. The control system includes a processor 126, which is connected to the detector 104, the illumination module 102, the guiding module 106 and the camera 107, such that the processor 126 can operate the detector 104, the illumination module 102, the shutter 121, the guiding module 106 and the camera 107, e.g. to turn them on and off. The processor 126 receives information from the detector 104 and the camera 107. The processor 126 also connected to a user interface 130 and a memory 128, and outputs information to the user interface 130 based on the information from the camera 107 and/or detector 104, and the memory 128. The processor 126 is also connected to a transmitter 142, and outputs the measurements taken by the detector 104 to an external device (not shown) via the transmitter 142.

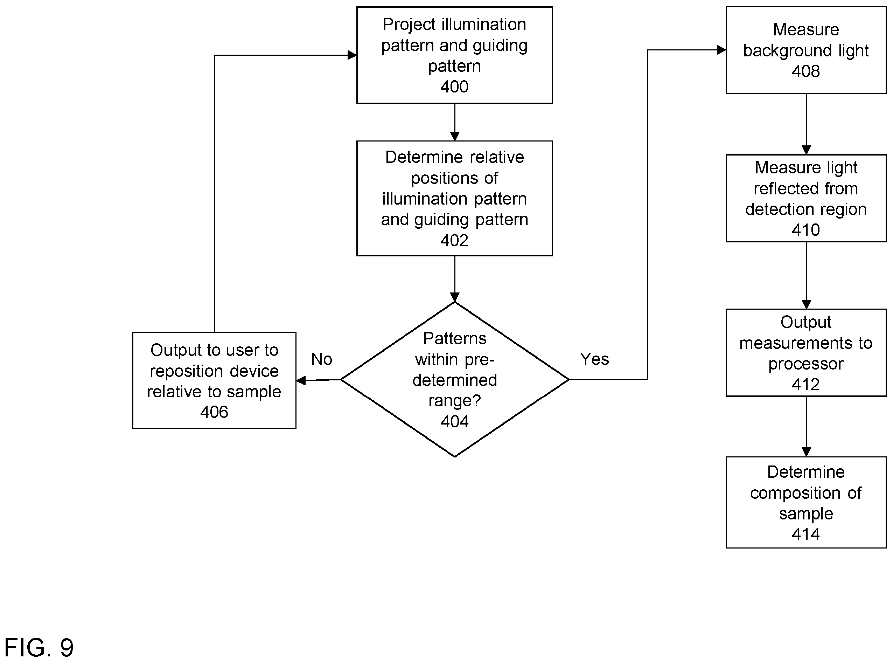

Figure 9 is a flow diagram of the method carried out by control system shown in Figure 8. At a first step 400, the processor 126 is in the 'guiding mode', and turns on the illumination module 102 and the guiding module 106 to project the illumination pattern and guiding pattern respectively onto the sample.

At a second step 402, the processor 126 turns on the camera 107, and receives an image from the camera of the relative positions of the illumination pattern and guiding pattern on the sample. The processor 126 then determines the relative positions of the illumination pattern and guiding pattern.

At a third step 404, the processor 126 determines whether the guiding pattern and the illumination pattern are in a predetermined range. If they are not in the predetermined range, at a fourth step 406 the processor 126 indicates to the user via the user interface 130 that the device needs to be moved relative to the sample. The processor 126 can indicate to the user via the user interface 130 whether the device 100 is 'too close' or 'too far' from the sample. As the determination of too far/close is dependent on the arrangement of the illumination and guiding modules in the device (as described with reference to Figures 3a-3c), the device may include a sensor (not shown) arranged to indicate to the user interface 130 whether the device is being held the right way up. This may avoid the user interface 130 indicating to the user to move the device 100 further away from the sample when it should be moved closer, because the device 100 is being held upside-down.

The processor 126 repeats the first, second and third steps 400, 402, 404 until the illumination pattern and the guiding pattern are within the predetermined range at the third step 404.

When the illumination pattern and the guiding pattern are in the predetermined range, the processor 126 moves from the 'guiding mode' to the 'illumination mode'. At a fifth step 408, the processor 126 turns on the detector 104 and turns off the illumination module 102 and the guiding module 106, and the detector 104 takes a measurement of the background light. The processor 126 receives the background light measurement from the detector 104 and stores it in the memory 128.

At a sixth step 410, the processor 126 turns on the illumination module 102 and the detector 104, and the detector 104 takes near infrared interactance spectroscopy measurements of the light from the illumination module 102 that is scattered inside the sample, at the point where the guiding pattern is/was projected onto the sample.

The processor 128 may also turn on the guiding module 106 at the sixth step 410, and the camera 107 may continue to output images of the illumination pattern and the guiding pattern to the processor 128. This allows the processor 128 to continue to compare the relative positions of the illumination pattern and the guiding pattern in the 'illumination mode', and output an indication via the user interface 130 that the device has moved relative to the sample.

At a seventh step 412, the detector 104 outputs the spectroscopy measurements of the sample to the processor 126, where the processor stores them in the memory 128.

At an eight step 414, the processor 126 outputs the measurements taken by the detector to an external unit via the transmitter 132 to determine the composition of the sample, or the processor 126 compares the measurements to data stored in the memory 128 to determine the composition of the sample. For example, the processor 126 may output an indication of the composition of the sample to the user interface 130.

The skilled person will appreciate that the steps described with reference to Figure 9 may also be carried out by a user instead of a camera and processor. For example, the user can visually determine at the second step 402 and the third step 404 whether the illumination pattern and guiding patterns are within the predetermined range (e.g. the fourth step 406 does not occur). The user can the input to the device to start taking measurements, where the processor can then execute the fifth, sixth, seventh and eight steps 408, 410, 412, 414.

Figure 10 shows example near-infrared interactance spectra for three different crab legs with differing meat contents, that could be measured by the detector 104 of the device 100 shown in Figure 1. The wavelength of the light detected by the detector 104 is shown on the x-axis, and the standard normal variate (SNV) normalised intensity of light absorption is shown on the y-axis.

The dashed line shows the example NIRS spectrum for a crab leg with a 95% meat content, the dotted line shows the example NIRS spectrum for a crab leg with a 75% meat content, and the solid line shows the example NIRS spectrum for a crab leg with a 40% meat content.

The detector 104 measures the NIRS spectrum for each crab leg, and in particular detects scattered light between approximately 400 nm and 1100 nm, and in particular detects a high intensity peak at 980 nm. These spectra are either output to an external device, or locally processed by the device 100.

For example, where the device 100 locally processes the spectra, the processor 126 compares the detected spectra to information stored in the memory 128 (e.g. example spectra), to identify that the peak at 980 nm is a water absorption peak, and the 980 nm peak decreases in intensity for a higher meat content. The percentage meat content of the crab legs is then output to the user via the user interface 130.

It will be appreciated by those skilled in the art that the invention has been illustrated by describing one or more specific embodiments thereof, but is not limited to these embodiments; many variations and modifications are possible, within the scope of the accompanying claims.

## Claims

1. A device for determining the composition of a sample, the device comprising:
an illumination module comprising a first light source arranged to project an illumination pattern on the sample in a first direction;
a detector arranged to measure light from the first light source scattered inside the sample into a field-of-view of the detector;
a guiding module comprising a second light source arranged to project a guiding pattern on the sample in a second direction;
wherein the guiding module is arranged to project the guiding pattern in the second direction at an angle relative to the first direction in which the illumination module projects the illumination pattern, such that the relative positions of the guiding pattern and the illumination pattern on the sample vary depending on the position of the device relative to the sample;
wherein the detector is positioned at an angle relative to the illumination module, such that the relative positions of the field-of-view of the detector and the illumination pattern on the sample vary depending on the position of the device relative to the sample;
wherein the guiding module, the illumination module and the detector are arranged such that the position of the guiding pattern projected onto the sample selectively indicates when the device is positioned relative to the sample such that the field-of-view of the detector and the illumination pattern are within a first predetermined range;
the device further comprising:
a processor programmed to:
operate the illumination module to project the illumination pattern;
operate the guiding module to project the guiding pattern;
operate the detector to take measurements;
receive measurements from the detector; and
output or store data representative of the measurements from the detector.

2. The device as claimed in claim 1, wherein the relative positions of the guiding pattern and the illumination pattern indicate that the device is positioned relative to the sample such that the field-of-view of the detector and the illumination pattern are within the first predetermined range on the sample, when the relative positions of the guiding pattern and the illumination pattern are within a second predetermined range on the sample.

3. The device as claimed in claim 2, wherein the device is arranged to receive an input from a user to take measurements when the user determines that the relative positions of the guiding pattern and the illumination pattern are within the second predetermined range.

4. The device as claimed in any preceding claim, comprising a camera arranged to image the guiding pattern;
wherein the processor is further programmed to:
receive an image from the camera; and
determine the position of the guiding pattern on the sample using the image from the camera.

5. The device as claimed in claim 4, wherein the processor is programmed to determine the position of the guiding pattern on the sample using the image from the camera, wherein the position of the guiding pattern on the sample indicates that the device is positioned relative to the sample such that the field-of-view of the detector and the illumination pattern are within the first predetermined range on the sample, when the position of the guiding pattern is within a predetermined position in the image captured by the camera.

6. The device as claimed in claim 4 or 5, wherein the camera is arranged to image the illumination pattern and the guiding pattern, and the processor is programmed to determine the relative positions of the illumination pattern and the guiding pattern on the sample using the image from the camera, wherein the relative positions of the guiding pattern and the illumination pattern indicate that the device is positioned relative to the sample such that the field-of-view of the detector and the illumination pattern are within the first predetermined range to the sample when the relative positions of the guiding pattern and the illumination pattern are within the second predetermined range on the sample.

7. The device as claimed in claim 6, wherein the processor is programmed to model the path of light through the sample using the relative positions of the illumination pattern and the guiding pattern on the sample, to determine the penetration of light into the sample.

8. The device as claimed in any of claims 4-7, wherein the processor is programmed to determine the stability of the measurements taken by comparing the light intensity of the measurements and the processor is programmed to output or store data representative of the measurements of stability optionally wherein the processor is programmed to determine discard the unstable measurements when the processor determines that the measurements are below a threshold stability.

9. The device as claimed in any preceding claim, wherein the field-of-view of the detector is adjacent to and spaced from the illumination pattern on the sample, when the field-of-view of the detector is within the first predetermined range from the illumination pattern on the sample.

10. The device as claimed in any preceding claim, wherein the detector comprises a spectrometer, wherein the detector is arranged to take measurements of the wavelengths and intensities of light scattered inside the sample;
optionally wherein the spectrometer is arranged to perform near-infrared interactance spectroscopy (NIRS);
further optionally wherein the processor is programmed to determine the composition of the sample, based on the data measured by the detector, and output an indication of the composition of the sample.

11. The device as claimed in any preceding claim, wherein the illumination module comprises a mask, wherein the mask is arranged to partially block the light projected by the first light source;
optionally wherein the illumination module comprises a doughnut-shaped mask arranged to a produce a ring-shaped illumination pattern.

12. The device as claimed in any of claims 4-11, comprising a user interface connected to the processor, wherein the user interface is arranged to indicate to the user when the device is positioned relative to the sample such that the field-of-view of the detector and the illumination pattern are within the first predetermined range;
optionally wherein the user interface is arranged to feedback to the user to move the device relative to the sample when the guiding pattern does not indicate that the device is positioned relative to the sample such that the field-of-view of the detector and the illumination pattern are within the first predetermined range;
further optionally wherein the user interface is arranged to provide continuous feedback of the position of the device relative to the sample.

13. The device as claimed in any of claims 4-11, wherein the device is arranged to be mounted to a robot;
move the device relative to the sample when the guiding pattern does not indicate that the device is positioned relative to the sample such that the field-of-view of the detector and the illumination pattern are within the first predetermined range.

14. A computer-implemented method for determining the composition of a sample using a device comprising a processor, an illumination module, a guiding module, a detector and a camera, wherein the processor is programmed to control the illumination module, the guiding module, the detector and the camera;
wherein the processor is programmed to, when operating in a guiding mode:
operate the illumination module to project an illumination pattern on the sample in a first direction;
operate the guiding module to project a guiding pattern on the sample in a second direction at an angle relative to the first direction in which the illumination module projects the illumination pattern, such that the relative positions of the guiding pattern and the illumination pattern vary depending on the position of the device relative to the sample;
receive an image from the camera of the illumination pattern and the guiding pattern; and
determine the position of the guiding pattern on the sample;
wherein the processor is programmed to, when the processor is operating in an illumination mode:
operate the detector to measure light scattered inside the sample into a field-of-view of the detector;
receive measurements from the detector; and
output or store data representative of the measurements from the detector;
wherein the processor is programmed to switch from the guiding mode to the illumination mode when the processor determines that the guiding pattern is positioned relative to the sample such that the field-of-view of the detector and the illumination pattern are within a first predetermined range.

15. A positioning module for a device for determining the composition of a sample, comprising:
an illumination module comprising a first light source arranged to project an illumination pattern on the sample in a first direction;
a guiding module comprising a second light source arranged to project a guiding pattern on the sample in a second direction;
wherein the guiding module is arranged to project the guiding pattern in the second direction at an angle relative to the first direction in which the illumination module projects the illumination pattern, such that the relative positions of the guiding pattern and the illumination pattern on the sample vary depending on the position of the device relative to the sample;
wherein the illumination module and the guiding module are arranged such that the relative positions of the guiding pattern and the illumination pattern projected onto the sample selectively indicates when the device is positioned relative to the sample such that the relative positions of the guiding pattern and the illumination pattern are within a predetermined range on the sample.
